Europäisches Patentamt

European Patent Office

Office européen des brevets

(19)

(11) Veröffentlichungsnummer: **0 257 443 B1**

# (12) EUROPÄISCHE PATENTSCHRIFT

(45) Veröffentlichungstag der Patentschrift: **13.05.92**

(51) Int. Cl.5: **C07C 209/16**, C07C 211/07

(21) Anmeldenummer: **87111648.9**

(22) Anmeldetag: **12.08.87**

(54) **Verfahren zur Herstellung von Trialkylaminen.**

(30) Priorität: **14.08.86 DE 3627592**

(43) Veröffentlichungstag der Anmeldung:
**02.03.88 Patentblatt 88/09**

(45) Bekanntmachung des Hinweises auf die
Patenterteilung:
**13.05.92 Patentblatt 92/20**

(84) Benannte Vertragsstaaten:
**BE DE ES FR GB IT NL**

(56) Entgegenhaltungen:
**EP-A- 0 009 590     EP-A- 0 227 904**
**DE-A- 2 709 864     DE-A- 3 246 978**
**DE-B- 2 057 001     DE-B- 2 114 614**

(73) Patentinhaber: **BASF Aktiengesellschaft**
**Carl-Bosch-Strasse 38**
**W-6700 Ludwigshafen(DE)**

(72) Erfinder: **Fischer, Roman, Dr.**
**Deidesheimer Strasse 1**
**W-6704 Mutterstadt(DE)**
Erfinder: **Mueller, Herbert, Dr.**
**Carostrasse 53**
**W-6710 Frankenthal(DE)**

**Beschreibung**

Die Erfindung betrifft ein Verfahren zur Herstellung von Trialkylaminen durch Umsetzung von primären Alkoholen mit Ammoniak in Gegenwart eines Hydrierungs-/Dehydrierungskatalysators.

Die katalytische Alkylierung von Aminen mit Alkoholen ist lange bekannt. Bei dieser Reaktion werden einerseits dehydratisierende Oxide, z.B. die des Aluminiums, Thoriums, Wolframs und Chroms als Katalysatoren verwendet, andererseits sind Hydrierungs- bzw. Dehydratisierungskatalysatoren, etwa auf der Grundlage von Kupfer, Nickel, Kobalt und Chrom, empfohlen worden. Es sind Verfahren in flüssiger Phase sowie in der Gasphase bekannt. In einer Arbeit von V.A. Nekrasova und N.I. Shuikin in der Zeitschrift "Russian Chemicals Reviews", 34, 843 (1965) sowie in dem Buch "The Acyclic Aliphatic Tertiary Amines", L. Spialter und J.A. Pappalardo, The Macmillian Comp., 1965, ist eine ausführliche Darstellung des Sachgebietes abgehandelt. In einer neueren Arbeit von A. Baiker und Jacek Kijenski wurde in der Zeitschrift "Catal. Rev.- Sci. Eng.", 27 (4), 653-697 (1985) nochmals eingehend berichtet.

Tertiäre Amine werden durch Alkylierung von Ammoniak, primären Aminen oder sekundären Aminen mit Alkoholen hergestellt. Als Folge von Transalkylierungs-/Disproportionierungsreaktionen enthalten die Reaktionsausträge neben den gesuchten tertiären Aminen sekundäre und primäre Amine oftmals in hohen Konzentrationen als Nebenprodukte. Besonders schwierig gestaltet sich die Herstellung der tertiären Amine aus Ammoniak und Alkoholen. Marktgängige tertiäre Amine wie Triethylamin, Tripropylamin, Tributylamin oder Trioctylamin werden industriell durchweg aus Ammoniak und den entsprechenden Alkoholen hergestellt. Man erhält die tertiären Amine als Koppelprodukte zusammen mit primären und sekundären Aminen. Bei der Umsetzung ist man darauf angewiesen, Ammoniak im Überschuß mit der Alkoholkomponente zur Reaktion zu bringen, damit die Aldolkondensation der intermediär gebildeten Carbonylverbindungen, die sich von den Alkoholen ableiten, in Grenzen gehalten werden kann. Andererseits wirkt der Ammoniaküberschuß naturgemäß der Bildung der tertiären Amine entgegen, und entsprechendes gilt für den Einsatz von Aminen anstelle des Ammoniaks.

Soll ein größerer Bedarf an tertiären Aminen gedeckt werden, hilft man sich oftmals dadurch, daß in einer getrennten Stufe primäre oder besser sekundäre Amine einer Nachalkylierung mit Alkoholen unterworfen werden. Die DE-A-1 493 781 beschreibt ein solches Verfahren, bei dem mindestens stöchiometrische Mengen des sekundären Amins mit dem Alkohol umgesetzt werden. Es soll also das umzusetzende Amin in der Reaktionszone in wenigstens äquimolarer Menge mit dem Alkohol vorliegen. Es wird gezeigt, daß ein Überschuß an Alkohol eine schlechte Selektivität hinsichtlich der gebildeten tertiären Amine zur Folge hat. Es bestätigt sich hier auch das großtechnisch immer angewendete Prinzip, durch die im Überschuß angewendete Stickstoffkomponente hohe Selektivitäten zu erreichen. Zwar scheint man bei der Umsetzung von sekundären Alkoholen mit einer solchen Maßnahme eine gewisse Verbesserung der Selektivität für das Amin zu erreichen, der Umwandlungsgrad des Amins ist jedoch gering. Bei primären Alkoholen werden jedenfalls geringe Umsätze und eine schlechte Selektivität beobachtet, denn als Nebenprodukte entstehen nun Rückstände, die die Ausbeute vermindern.

Ein verbessertes Verfahren zur Herstellung von tertiären Aminen beschreibt US-A-3 708 539; dabei wird in flüssiger Phase ein Alkohol mit einem sekundären Amin an Katalysatoren auf der Grundlage von Ruthenium, Osmium, Rhenium oder Technetium zur Reaktion gebracht. Nachteil dieses Verfahrens ist der Umstand, daß Umsatz und Ausbeute, bezogen auf die eingesetzten Alkohole, gemessen am Wert der Katalysatorrohstoffe, für ein technisches Verfahren unzureichend sind.

Besonders tertiäre Amine vom Typ der Dimethylfettalkylamine, deren Fettalkylrest im allgemeinen eine C-Zahl > 8 aufweist, sind technisch interessant. Diese Produkte werden nach den verschiedensten Methoden, sei es in der Gasphase, sei es in der Flüssigphase, in großem Umfang hergestellt.

Größere Schwierigkeiten bereitet die Synthese der niedermolekularen tertiären Alkylamine.

Prototypen dieser Verbindungsklasse sind z.B. das Dimethylethylamin und Triethylamin. Diese tertiären Amine werden industriell in großem Umfang als Katalysatoren für die Beschleunigung der Polymerisation von Polyurethanharzen verwendet, z.B. in Kernsandbindemitteln. Weitere tertiäre Amine dieser Produktklasse sind z.B. n-Propyldimethylamin, Methylethyl-n-propylamin, Isopropyldimethylamin oder n-Butyldimethylamin. Alle Versuche, diese niedermolekularen, tertiären Amine nach der Methode herzustellen, die bei der Synthese der langkettigen Amine zum Erfolg führte, scheiterten bislang. Deshalb wurde in der DE-A-2 838 184 und in der EP-B-24 225 vorgeschlagen, die Synthese dieser Verbindungen entweder an Kupfer-Chromit-Katalysatoren oder reinen Kupfer-Katalysatoren, die durch thermische Zersetzung oder Reduktion von basischen Kupfer-Aluminium-Carbonaten entstehen, durch Reaktion in der Gasphase, z.B. aus Dimethylamin und den entsprechenden niedermolekularen Alkoholen herzustellen. Gesundheitliche Gründe sprechen aber gegen die Verwendung von Chromit-Katalysatoren, da diese cancerogen sind. Den Gasphasenverfahren haftet ganz allgemein der Nachteil an, daß für die Durchführung der Gasphasenreaktion ein

EP 0 257 443 B1

verhältnismäßig hoher Energieaufwand für das Verdampfen der Reaktionskomponenten und die Aufrechterhaltung eines ausreichend großen Gasstromes aufgewendet werden muß, um Kondensationen am Katalysator zu verhindern. Das Zielprodukt muß zudem durch großen Kondensationsaufwand aus der geförderten Kreisgasmenge abgeschieden werden. Meist sind dafür hintereinander mehrstufige Kondensationen erforderlich. Da diese Verfahren in der Gasphase bei relativ niedrigen Wasserstoffpartialdrücken an den nicht nur hydrierend, sondern auch dehydrierend wirkenden Katalysatoren durchgeführt werden, bildet sich aus dem Alkohol auch der entsprechende Aldehyd, der durch Aldolkondensation zur unerwünschten Alkylgruppe mit der verdoppelten C-Zahl und damit zu Nebenprodukten führt, welche nicht nur die Ausbeute mindern, sondern auch als Verunreinigungen im Endprodukt erscheinen. In der EP-B-24 225 wird z.B. so die Entstehung von Acetaldehyd aus Ethanol und die Bildung von $C_4$-Resten beschrieben.

In der US-A-4 480 131 wird ebenfalls ein Gasphasenverfahren zur Herstellung von sekundären und in geringerem Maß auch tertiären Aminen beschrieben. Primäre Amine werden durch Alkohole zu sekundären und tertiären Aminen alkyliert. Die Arbeitsweise mit einem Aminüberschuß wird bevorzugt. Trotzdem ist die Reaktion nicht sehr selektiv. Die Umsetzungen sind zudem unvollständig und man beobachtet, wie es die dort beschriebenen Beispiele durchweg zeigen, Disproportionierungsreaktionen in erheblichem Ausmaße, die bis zur Ammoniakbildung führen.

Man hat sich auch immer wieder bemüht, Ammoniak durch Alkylierung mit Alkoholen in Gegenwart eines Hydrierungs-/Dehydrierungskatalysators direkt in die tertiären Amine zu überführen. Die US-A-2 953 601 beschreibt die Umsetzung bei Normaldruck, wobei das Reaktionswasser kontinuierlich als azeotropes Gemisch mit dem überschüssigen Alkohol entfernt und zurückgewonnener Alkohol wieder in das Reaktionsgefäß geleitet wird. Unterläßt man die Ausschleusung des Reaktionswassers, so kommt die Umsetzung zum Stillstand. Nach der beschriebenen Arbeitsweise erhält man eine Ausbeute an tertiären Aminen von 58 bis 65 %. Der Anteil an primären und sekundären Aminen und Nebenprodukten ist jedoch erheblich.

Ein zweistufiges Verfahren nach der US-A 3 223 734 soll diese Nachteile vermeiden. In einem ersten Schritt wird ein gesättigter Alkohol mit 4 bis 22 C-Atomen mit Ammoniak in Gegenwart eines Hydrierungs-/Dehydrierungs-katalysators umgesetzt, wonach dem erhaltenen Gemisch aus primären, sekundären und tertiären Aminen sowie nicht umgesetztem Alkohol weiterer Alkohol zugesetzt wird, so daß es die zur stöchiometrischen Umsetzung zu tertiären Aminen erforderliche Alkoholmenge enthält. Es wird dann in einem zweiten Reaktionsschritt im Temperaturbereich von 190 bis 230 ° C in gleicher Weise behandelt wie in der ersten Stufe, wobei die in der ersten Stufe erhaltenen primären und sekundären Amine unter azeotroper Abführung des Reaktionswassers in tertiäre Amine umgewandelt werden. Es werden zwar Ausbeuten an tertiären Aminen bis zu 98 % angegeben, die analytische Überprüfung mittels Gaschromatographie zeigt jedoch, daß die Amine sehr stark verunreinigt sind. Sie können durch Destillation nicht genügend rein gewonnen werden. Man findet in den Aminen Alkylgruppen mit im Vergleich zu den eingesetzten Alkoholen verdoppelter C-Zahl. Außerdem fallen noch eine Reihe anderer Nebenprodukte wie Säureamide, sekundäre Amine und sonstige Stoffe an. Selbst nach sorgfältiger Destillation des erhaltenen Rohamingemisches gelingt es nicht, einen Reinheitsgrad zu erreichen, der beispielsweise von den Produkten bei der Anwendung auf dem Naßmetallurgiegebiet gefordert wird. Das Trennvermögen der Amine für Metallionen wird durch den Gehalt an Begleitstoffen wie Säureamiden, Nitrilen, Kohlenwasserstoffen und Acetalen) erheblich beeinträchtigt.

In der DE-A-2 057 001 ist ein Verfahren zur Herstellung von tertiären Aminen aus Ammoniak und primären Alkoholen in Gegenwart von Hydrierungs-/Dehydrierungskatalysatoren beschrieben. Bei dieser Arbeitsweise ist es erforderlich, das bei der Reaktion entstehende Wasser laufend zu entfernen. Man arbeitet unter Anwendung der Kreisgasfahrweise, was insofern nachteilig ist, als diese mit einem erhöhten thermischen und mechanischen Energieaufwand verbunden ist. Zwar erhält man nach der Arbeitsweise der DE-A-2 057 001 hohe Selektivitäten bei der Umsetzung von Alkoholen mit Ammoniak, die Aktivität der mit einem primären, sekundären oder tertiären Amin vorbehandelten Katalysatoren reicht noch nicht aus, daß man auf die laufende Entfernung des Reaktionswassers bei der Umsetzung verzichten könnte. Als Katalysatoren werden solche auf der Basis von Nickel, Kobalt oder Eisen empfohlen. Von besonderem Interesse ist der experimentelle Befund im Teil 2 des Beispiels 2 dieser DE-A, nach dem bei Abwesenheit von Ammoniak etwa die Hälfte des als Alkohol eingesetzten Isononanols durch den Katalysator in Isononanal-diisononanolacetal überführt wird. Dieser Befund spricht, insbesondere in Zusammenschau mit dem vorstehend besprochenen Stand der Technik, dafür, Ammoniak stets als Überschußkomponente einzusetzen, um eine derartige unerwünschte Reaktionen zu unterbinden.

In der DE-A 27 09 864 wird bei der Herstellung von tertiären aliphatischen Aminen aus Alkoholen und primären oder sekundären Aminen bei Temperaturen von 160 - 350 ° C und bei kontinuierlicher Ausführung bei einem Druck bis zu 10 bar gearbeitet, so daß das Reaktionswasser aufgrund dieser Reaktionsbedingungen sofort entweicht.

3

Bei dem in der DE-A 32 46 978 beschriebenen Verfahren zur Herstellung primärer, sekundärer oder tertiärer Amine durch Umsetzung von Ammoniak oder primären Aminen mit primären oder sekundären Alkoholen an einem Kupferkatalysator wird es zur Erzielung optimaler Ergebnisse als wichtig bezeichnet, Wasser laufend aus dem Reaktionsgemisch zu entfernen.

Auch nach dem Verfahren der US-A 4 404 404, das die Herstellung von Aminen aus aliphatischen Alkoholen oder aliphatischen Aldehyden mit Ammoniak oder einem primären Amin in flüssiger Phase in Gegenwart eines Katalysators aus Kupferoxid oder -hydroxid und einem Metalloxid oder -hydroxid der Gruppe IIA des Periodensystems beschreibt, wird das entstehende Reaktionswasser laufend aus dem Umsetzungsgemisch entfernt.

Eine Umsetzung in Anwesenheit des bei der Reaktion gebildeten Wassers ist in der EP-A 227 904 beschrieben, nach der Trialkylamine aus Dialkylaminen und Alkoholen in Gegenwart von Kupferkatalysatoren in flüssiger Phase dargestellt werden.

Der Erfindung lag die Aufgabe zugrunde, ein Verfahren der eingangs genannten Art zur Verfügung zu stellen, das die Nachteile des Standes der Technik nicht aufweist, die Herstellung sehr reiner tertiärer Amine in hoher Ausbeute ermöglicht und die notwendige Reinigung durch eine möglichst einfache Destillation erreichen läßt.

Demgemäß wurde ein Verfahren zur Herstellung von Trialkylaminen durch Umsetzung von primären Alkoholen mit Ammoniak oder einem primären Alkylamin in Gegenwart eines Hydrierungs-/Dehydrierungskatalysators sowie in Gegenwart von Wasserstoff gefunden, welches dadurch gekennzeichnet ist, daß man

a) die Reaktion kontinuierlich in flüssiger Phase bei einem Gesamtdruck von 50 bis 300 bar durchführt,

b) hierbei im Zulauf der Reaktionspartner einen stöchiometrischen Überschuß von 1 bis 15 mol des Alkohols über das Ammoniak bzw. das primäre Alkylamin einhält,

c) die Reaktion in Anwesenheit des bei der Reaktion gebildeten Wassers durchführt und

d) einen einen Hydrierungs-/Dehydrierungskatalysator einsetzt, der als katalytisch hydrierendes/dehydrierendes Metall im wesentlichen nur Kupfer enthält.

Das erfindungsgemäße Verfahren läßt sich in einfachen, unkomplizierten und deshalb billigen Reaktionsapparaten mit praktisch quantitativen Ausbeuten durchführen. Der hierbei erforderliche Energieaufwand ist gering. Das Rohgemisch des Reaktionsaustrages läßt sich in einfacher Weise zu den gewünschten reinen Zielprodukten aufarbeiten. Die einzusetzenden Kupferkatalysatoren sind billig und weisen eine sehr lange Lebensdauer auf. Beim erfindungsgemäßen Verfahren wird die Bildung unerwünschter Nebenprodukte unterdrückt, und man erhält die Wertprodukte mit hoher Selektivität.

Obwohl das bei der Reaktion gebildete Wasser im wesentlichen im Reaktionsansatz verbleibt, wird hierdurch weder die Ausbeute an den Verfahrensprodukten vermindert noch nicht die Reaktionsgeschwindigkeit, vielmehr nehmen Ausbeute und Reaktionsgeschwindigkeit unerwarteterweise zu.

Nicht vorhersehbar, für das erfindungsgemäße Verfahren aber von ausschlaggebender Bedeutung war es, daß im Gegensatz zum Verfahren der DE-A-2 075 001 bei Einhaltung der erfindungsgemäßen Reaktionsparameter, der dehydrierende Einfluß des Katalysators auf den Alkohol auch bei Abwesenheit von Ammoniak unterbleibt. Während unter den vergleichbaren Verfahrensbedingungen der DE-A-2 057 001 etwa die Hälfte des Alkohols in Nebenreaktionen verbraucht wird, findet ein derartiger Verlust unter den erfindungsgemäßen Reaktionsbedingungen nicht statt.

Die hohe Selektivität für die Bildung der tertiären Amine gemäß dem erfindungsgemäßen Verfahrens steht im Gegensatz zu dem Ergebnis, über das von A. Baiker und J. Kijenski in der oben zitierten Literaturstelle berichtet wird. Danach entsteht aus Ammoniak und Octanol an Kupferkatalysatoren bei 240°C ein Reaktionsaustrag, der 21 Gew.% Octanol, 25 Gew.% Octylamin, 30 Gew.% Dioctylamin, 7 Gew.% Trioctylamin, 14 Gew.% Caprylonitril und 3 Gew.% andere Nebenprodukte enthält. Eine ähnliche Produktverteilung wird auch bei der Umsetzung von Dodecanol bzw. Decanol erhalten (siehe Seite 690 der Druckschrift).

Vergleicht man das erfindungsgemäße Verfahren mit den Ergebnissen der Aminierung von Ethanol mit Ammoniak zu Ethylaminen wie es in EP-B-13 176 mitgeteilt wird, so überrascht die hohe Selektivität bezüglich der tertiären Amine, die man nach dem erfindungsgemäßen Verfahren bei Einhaltung der vorgeschriebenen Reaktionsparameter erhält. In der EP-B-13 176 werden Alkohol und Ammoniak im wesentlichen zum primären Amin umgesetzt.

Man verwendet beim erfindungsgemäßen Verfahren Katalysatoren, die als aktive Komponente im wesentlichen nur Kupfer enthalten, und zwar entweder als Festbett oder in suspendierter Form. Man bringt die Reaktionspartner zweckmäßig in Gegenwart von in flüssiger Phase gelöstem oder suspendiertem Alkali- und/ oder Erdalkalioxid und/oder -hydroxid zur Umsetzung. Man kann als Alkali- bzw. Erdalkalioxid bzw. -hydroxid, Oxide bzw. Hydroxide des Lithiums, Natriums, Kaliums, Magnesiums, Calciums, Strontiums, Bariums oder Mischungen hiervon einsetzen.

Der Gesamtdruck beträgt 50 bis 300, vorzugsweise 50 bis 200 bar, wovon etwa 10 bis 50 % auf den Wasserstoffpartialdruck entfallen. Bei diesen Drücken bilden die Reaktionspartner eine flüssige Phase.

Im Gegensatz zu anderen, früher beschriebenen Verfahren ist es erfindungsgemäß nicht notwendig, das bei der Umsetzung gebildete Wasser aus dem Reaktionsgemisch laufend zu entfernen, um eine vollständige Umsetzung des Ammoniaks zu erreichen. Besonders dieses letztere Ergebnis war bei der Umsetzung in flüssiger Phase nicht zu erwarten, da es aus vielen anderen Fällen bekannt ist (vgl. z.B. DE-C-2 625 196), daß die vollständige Alkylierung nur gelingt, wenn Wasser im Maße seiner Bildung aus dem Reaktionsgemisch entfernt wird.

Nach dem erfindungsgemäßen Verfahren können insbesondere einwertige aliphatische Alkohole mit der Kohlenstoffzahl 2 bis 24 wie Ethanol, n-Propanol, i-Propanol, n-Butanol, n-Heptanol, n-Dodecanol oder z.B. Stearylalkohol, gegebenenfalls in Gegenwart von Wasserstoff, bei nahezu quantitativer Ausbeute mit Ammoniak zu den tert. Trialkylaminen umgesetzt werden. Anstelle der reinen Alkohole können auch Alkoholgemische umgesetzt werden.

Schließlich sei auf die Möglichkeit hingewiesen, die Reaktion mit einem bestimmten Alkohol zu beginnen und bevor die Alkylierung des Ammoniaks vollständig vollzogen ist, einen weiteren Alkohol, der vom zuerst eingesetzten Alkohol verschieden ist, dem Reaktionsgemisch zuzusetzen. Man erreicht dadurch eine bestimmte Alkylgruppenverteilung am tertiären Amin. Obwohl es bekannt ist, daß Kupferkatalysatoren die Disproportionierung der Alkylamine sehr stark katalysieren, gelingt es durch Einhaltung der erfindungsgemäßen Parameter, insbesondere durch Zugabe der oben genannten alkalischen Substanzen in der flüssigen Phase, diese Disproportionierungsreaktion weitgehend zu vermeiden. Die Zugabe solcher alkalisierenden Substanzen zu den Festkatalysatoren, wie es z.B. in der EP-B-13 176 beschrieben ist, verhütet nicht die disproportionierende Wirkung der Kupferkatalysatoren. Die durch Kupferkatalysator bewirkte Disproportionierung der Alkylamine wird in der Literaturstelle "Ind. Eng. Chem. Prod. Res. Dev.", Vol. 22, No. 2, 1983, Seite 219 abgehandelt. Bei der Ausübung des erfindungsgemäßen Verfahrens wird die Bildung von primären Aminen höchstens in Spuren und die von sek. Aminen nur in untergeordneten Mengen beobachtet.

Das Verfahren wird kontinuierlich in der Flüssigphase an Festbettkatalysatoren nach der sogenannten Sumpf- oder Rieselfahrweise oder auch an suspendierten Katalysatoren ausgeübt.

An Festkatalysatoren arbeitet man zweckmäßig folgendermaßen:

Als Reaktor verwendet man z.B. ein senkrecht angeordnetes zylindrisches Gefäß mit den üblichen Einrichtungen zur Kühlung oder Heizung und der Zufuhrmöglichkeit der Reaktionsteilnehmer. Das Reaktionsgefäß ist mit dem Katalysator, der beliebig geformt sein kann, gefüllt. Häufig verwendet man den Katalysator als Strangpressling mit einem Durchmesser von 3 bis 6 mm und einer Länge bis zu 6 cm. Er kann aber auch tablettiert in Form von Zylindern mit den Maßen von z.B. 5 mm Durchmesser und 5 mm Höhe oder als Kugel oder in jeder anderen beliebigen Form eingesetzt werden.

Wendet man die Rieselfahrweise an, so wird das den Katalysator enthaltende Reaktionsgefäß von oben nach unten mit den Reaktionspartnern Ammoniak und Alkohol beaufschlagt, z.B. mit einer Geschwindigkeit von 200 bis 500 ml Reaktionsgemisch pro Liter Katalysatorvolumen und Stunde, und man stellt den gewünschten Reaktionsdruck, der sich aus dem Dampfdruck der Reaktionspartner und Reaktionsprodukte, dem Wasserstoffpartialdruck und gegebenenfalls zusätzlich verwendeten Inertgasdruck zusammensetzt, ein.

Auf den Auslaß von Abgasen kann weitgehend verzichtet werden, da sich nach dem erfindungsgemäßen Verfahren fremde Inertgase praktisch nicht bilden. Insofern ist das Verfahren im Bezug auf den Wasserstoffverbrauch besonders ökonomisch. Die bei den meisten Aminierungsverfahren beobachtete Decarbonylierung der aus dem Alkohol intermediär gebildeten Carbonylverbindung findet im erfindungsgemäßen Verfahren praktisch nicht statt. beobachtet.

Das die Reaktionszone verlassende Produkt gelangt in eine standgeregelte Vorlage, aus der das Produktgemisch vorzugsweise diskontinuierlich entnommen wird. Da die Reaktion mit ca. 14 kcal/Mol leicht exotherm ist, kann das Reaktionsgefäß isotherm gehalten werden, indem man einen Teil des Umsetzungsproduktes mittels einer Kreispumpe nach vorhergehender Kühlung auf die Katalysatorschüttung zurückführt. Man führt die Reaktion zweckmäßig so durch, daß der eingesetzte Ammoniak vollständig umgesetzt wird. Man erspart sich dadurch Investitionen und den Betrieb einer kostenmäßig aufwendigen Ammoniakrückführungskolonne. Vielmehr gewinnt man das reine Trialkylamin durch eine einfache destillative Trennung vom im Überschuß eingesetzten Alkohol, der in die Umsetzung zurückgebracht wird. Da es der Umsetzung nicht schadet, wenn zusammen mit dem zurückgeführten Alkohol geringe Mengen Reaktionswasser in die Umsetzung mit eingetragen werden, gestaltet sich die destillative Aufarbeitung im erfindungsgemäßen Verfahren äußerst einfach.

Obwohl in technischer Hinsicht von untergeordneter Bedeutung, kann in einer Variante des erfindungsgemäßen Verfahrens statt Ammoniak auch ein primäres, vor allem niedermolekulares Amin mit einer C-Zahl von 1 bis 6, für die Herstellung der tertiären Trialkylamine eingesetzt werden. Diese Umsetzung hat besonders Bedeutung für die Synthese von gemischt alkylierten tertiären Aminen. Sie ist vorteilhaft im Vergleich zu allen übrigen bekannt gewordenen Verfahren, da keinerlei Alkyldisproportionierung beobachtet wird, sofern man in einem nicht allzu extremen Temperaturbereich (Reaktionstemperatur unterhalb von 240°C) arbeitet. Der Umsetzung von Ammoniak mit den Alkoholen wird aber die größere technische und ökonomische Bedeutung beigemessen.

Neben der oben beschriebenen und bevorzugten Anwendung der Rieselfahrweise läßt sich die Umsetzung auch dergestalt durchführen, daß die Reaktionspartner von unten nach oben in flüssiger Form das Katalysatorbett durchströmen (Sumpffahrweise). Auch hier kann Reaktionswärme gegebenenfalls durch Rückführung abgeführt werden. Wie bei der Rieselfahrweise ist es auch möglich, durch den im Überschuß eingesetzten Reaktionspartner die Reaktionsführung isotherm ablaufen zu lassen.

In einer bevorzugten Ausführungsform des erfindungsgemäßen Verfahrens verwendet man Kupferkatalysatoren, die in der DE-A-2 445 303 beschrieben worden sind. Sie können als amorphe Produkte der thermischen Zersetzung und Reduktion basischer Kupfer-Aluminiumverbindungen angesehen und dadurch erhalten werden, daß man verdünnte Lösungen von Kupfer- und Aluminiumsalzen, deren Molarität zweckmäßigerweise unter 3 liegt, mit einem Alkalicarbonat bei pH 8 bis 10 versetzt und die erhaltenen Niederschläge vor oder nach entsprechender Formung bei einer Temperatur von 350 bis 600°C zersetzt. Nach üblicher Reduzierung, bevorzugt in Gegenwart des bei der späteren Umsetzung verwendeten Alkohols, erhält man hochaktive, für das vorliegende Verfahren bestens geeignete Katalysatoren. Es können aber auch Kupferkatalysatoren verwendet werden, die man durch Tränken von geeignetem Trägermaterial, z.B. Bimsstein, Diatomeenerde, Kieselgel, Thorium- oder Aluminiumoxid, sowie nachfolgende Reduktion erhält.

Bei der nach dem erfindungsgemäßen Verfahren ebenfalls möglichen Suspensionsfahrweise ist der reduzierte Kupferkatalysator in den Reaktionskomponenten Alkohol, Ammoniak oder Monoalkylamin suspendiert. Geeignete Katalysatoren sind z.B. Raney-Kupfer oder die oben beschriebenen Kupferkatalysatoren in gepulverter Form. Bevorzugt ist jedoch ein aktives Kupfermaterial, das man dadurch erhält, daß man Kupferformiat in Gegenwart eines Alkohols und eines Dialkylamins auf 200 bis 250°C erhitzt. Die Bildungsweise eines solchen Katalysators ist z.B. in der EP-B-70 512 beschrieben.

Die erfindungsgemäße Synthese der tertiären Amine wird in Gegenwart eines stöchiometrischen Überschußes an Alkohol über Ammoniak bzw. Amine vorgenommen, wobei der Überschuß definitionsgemäß mindestens etwa 1 Mol betragen soll. Im Falle des Ammoniaks liegt das Molverhältnis Alkohol/Ammoniak also zwischen 4:1 und 18:1, und im Falle der primären Amine liegt dieses Verhältnis zwischen 3:1 und 17:1, wobei allerdings die oberen Grenzen nicht kritisch sondern lediglich durch die Wirtschaftlichkeit gegeben sind. Die optimalen Überschüsse können durch einige Versuche leicht ermittelt werden, wobei allgemein die Regel gilt, daß der Überschuß mit steigendem Molekulargewicht des Alkohols zunimmt.

Eine sehr zweckmäßige Maßnahme beim vorliegenden Verfahren besteht darin, den flüssigen Reaktionspartnern ein Erdalkali- und/oder Alkalioxid oder -hydroxid zuzugeben.

Durch diese Maßnahme gelingt es, Umalkylierungsreaktionen und Disproportionierungen praktisch völlig zu unterdrücken. Dabei erzielt man in der Regel die beste Wirkung, wenn diese Basen im Reaktionsgemisch gelöst sind, jedoch genügt auch eine Suspension. Ein deutlich geringerer Effekt ist bei Verwendung von Kupferkatalysatoren zu beobachten, die mit den Basen dotiert sind, d.h. die Basen sollten möglichst in freier Form vorliegen.

Die Reaktion wird zweckmäßig in einem Temperaturbereich von 190 bis 280°C durchgeführt. Der bevorzugte Bereich beträgt 200 bis 240°C.

Die Reaktionsgeschwindigkeit ist sehr hoch, es lassen sich ohne weiteres stündlich 100 bis 600 Gew.-Teile tert. Trialkylamin pro Liter Katalysatorvolumen erzeugen.

Bei der Durchführung des erfindungsgemäßen Verfahrens stellte man in überraschender Weise - weil bei Aminierungen nicht bekannt - fest, daß die Katalysatoren über außerordentlich lange Zeiträume ihre Aktivität unvermindert beibehalten. In der Festbettkatalyse z.B. kann an dem bevorzugten Kupferkatalysator, gemäß der DE-A-2 445 303, mehr als ein Jahr gearbeitet werden.

Beispiel 1

Für die kontinuierliche Herstellung des Triethylamins wurde aus 7309 g Ethanolazeotrop (95,6 Gew.% Ethanol, 4,4 Gew.% Wasser mit 1,2 Gew.% Toluol als Vergällungsmittel) und 289 g Ammoniak unter Zusatz von 0,025 Gew.% Natriumhydroxid als 50 %ige Lösung eine Zulaufmischung hergestellt.

Die Aminierung wurde in einem senkrecht stehenden Reaktionsrohr mit 1000 ml Reaktionsinhalt durchgeführt. Das Verhältnis des Durchmessers zur Länge des Reaktionsrohres betrug 1:40. Mittels eines organischen Wärmeträgers, der in einem Heizmantel umgepumpt wurde, ließ sich das Reaktionsrohr thermostatisieren. In das Reaktionsrohr waren 700 ml einer Katalysatorschüttung gefüllt. Die Katalysatorteilchen hatten die Form von Zylindern, die 3 mm lang waren und einen Durchmesser von 3 mm hatten. Der Katalysator war nach der Vorschrift des Beispieles 1 der DE-A-2 445 303 hergestellt worden. Er wurde bei 180 bis 200°C mit Wasserstoff reduziert, wobei gleichzeitig stündlich 300 ml Ethanol über den Katalysator gerieselt wurden. Die Reduktion des Katalysators wurde zunächst mit einem Wasserstof/Stickstoff-Gemisch (50 bar), später mit reinem Wasserstoff durchgeführt, bis im abfließenden Ethanolazeotrop kein Reduktionswasser mehr nachweisbar war. Dem Ofen wurden dann bei 250°C stündlich 300 g des oben beschriebenen ammoniakhaltigen Zulaufes bei 200 bar Gesamtdruck (Wasserstoffpartialdruck < 150 bar) von oben nach unten zugefahren. Am Kopf der Vorlage wurden stündlich 900 ml (Normaldruck) Wasserstoff als Abgas gefahren. Das die Vorlage verlassende Reaktionsprodukt hatte nach gaschromatographischer Analyse und Destillationsanalyse wasserfrei gerechnet folgende Zusammensetzung:

| | |
|---|---|
| Diethylamin | < 0,5 Gew.% |
| Triethylamin | 23 Gew.% |
| Ethanol | 76 Gew.% |
| Unbekannte und Rückstand | < 0,5 Gew.% |

Durch Destillation läßt sich aus diesem Rohgemisch Triethylamin mit über 99,9 % Reinheit gewinnen.

Ein ähnliches Ergebnis wurde mit den gleichen molaren Mengen an Kaliumhydroxid und Lithiumhydroxid anstelle des Natriumhydroxids erzielt.

Bei Verwendung eines Kupfertränkkatalysators (20 Gew.% Cu auf Kieselgel) und bei einem Zulauf der Reaktionspartner von rd. 150 g/h wurde ebenfalls praktisch das gleiche Ergebnis erzielt.

Beispiel 2

Für die Synthese von Tri-n-pentylamins stellte man eine Lösung von 1 mol n-Pentylamin in 4 mol n-Pentanol her. Diese Lösung wurde mit 0,25 Gew.% 50 gew.%iger Natronlauge versetzt.

Die Aminierung wurde in der im Beispiel 1 beschriebenen Apparatur durchgeführt. Die Reaktionstemperatur betrug 230 bis 235°C und der Gesamtdruck 60 bar, wovon rd. 10 bar auf den Wasserstoffpartialdruck entfielen. Die Zulaufmenge der Reaktionskomponenten pro Stunde wurde bei 300 ml und die Abgasmenge bei 500 ml gehalten.

Das erhaltene Reaktionsprodukt wurde einer Destillationsanalyse unterworfen. Danach setzte sich der Reaktionsaustrag wie folgt zusammen (Reaktionswasser nicht berücksichtigt):

| | |
|---|---|
| n-Pentanol | 51 Gew.% |
| Tri-n-pentylamin | 45 Gew.% |
| Di-n-pentylamin | 3 Gew.% |
| Gesamtunbekannte | 0,8 Gew.% |
| Hochsieder | 0,2 Gew.% |

Durch fraktionierte Destillation gewann man das Tripentylamin in über 99,5 %iger Reinheit.

Beispiel 3

Man stellte zunächst eine Lösung aus 8 Mol n-Butanol und 1 Mol Ammoniak bei Raumtemperatur her. Diese Lösung wurde mit 0,05 Gew.% 50 gew.%iger Natronlauge versetzt.

Für die Aminierung verwendete man die im Beispiel 1 beschriebene Apparatur und führte die Aminierung bei 230°C mit 300 g Zulauf pro Stunde durch. Der Gesamtdruck bei der Synthese betrug 200 bar und der Wasserstoffpartialdruck 120 bar, die stündliche Abgasmenge 500 ml bei Normaldruck. Man erhielt ein Reaktionsprodukt, das nach der gaschromatographischen Analyse und einer Destillationsanalyse folgende Zusammensetzung - wasserfrei gerechnet - aufwies:

| | |
|---|---|
| Tri-n-butylamin | 28 Gew.% |
| Di-n-butylamin | 1 Gew.% |
| n-Butanol | 71 Gew.% |

An unbekannten Nebenprodukten und Höhersiedern waren weniger als 0,4 Gew.% entstanden.

Setzte man unter denselben Reaktionsbedingungen n-Octanol mit Ammoniak im Molverhältnis 7:1 um, so erhielt man das Tri-n-octylamin mit über 95 mol.% Ausbeute. Gleichzeitig gebildetes 2,5 % Di-n-octylamin (2,5 Gew.%) ließ sich aus dem Reaktionsaustrag quantitativ abtrennen, dem Frischzulauf zusetzen und mit Octanol alkylieren. Der dabei entstehende Reaktionsaustrag enthielt das sekundäre Amin ebenfalls etwa mit 2 Gew.%, so daß im stationären Zustand, bei dem das entstandene sekundäre Amin immer wieder zurückgeführt wurde, die Umsetzung zum tertiären Amin praktisch quantitativ ablief.

**Patentansprüche**

1. Verfahren zur Herstellung von Trialkylaminen durch Umsetzung von primären Alkoholen mit Ammoniak oder einem primären Alkylamin in Gegenwart eines Hydrierungs-/Dehydrierungskatalysators sowie in Gegenwart von Wasserstoff, dadurch gekennzeichnet, daß man

   a) die Reaktion kontinuierlich in flüssiger Phase bei einem Gesamtdruck von 50 bis 300 bar durchführt,

   b) hierbei im Zulauf der Reaktionspartner einen stöchiometrischen Überschuß von 1 bis 15 mol des Alkohols über das Ammoniak bzw. das primäre Alkylamin einhält,

   c) die Reaktion in Anwesenheit des bei der Reaktion gebildeten Wassers durchführt und

   d) einen Hydrierungs-/Dehydrierungskatalysator einsetzt, der als katalytisch hydrierendes/dehydrierendes Metall im wesentlichen nur Kupfer enthält.

2. Verfahren nach Anspruch 1, dadurch gekennzeichnet, daß man die Reaktionspartner in Gegenwart von Alkali- und/oder Erdalkalioxiden und/oder -hydroxiden umsetzt.

3. Verfahren nach Anspruch 2, dadurch gekennzeichnet, daß man die Alkali- und/oder Erdalkalioxide und/oder -hydroxide in einer Menge von 0,01 bis 1, insbesondere 0,02 bis 0,2 Gew.%, bezogen auf die Menge der Reaktionspartner, einsetzt.

4. Verfahren nach den Ansprüchen 1 bis 3, dadurch gekennzeichnet, daß man $C_1$-$C_{24}$-Alkohole in reiner Form oder in Form ihrer Mischungen einsetzt.

5. Verfahren nach den Ansprüchen 1 bis 4, dadurch gekennzeichnet, daß der stöchiometrische Überschuß des Alkohols über Ammoniak bzw. Amin 1,5 bis 15, vorzugsweise 2 bis 10 mol beträgt.

6. Verfahren nach den Ansprüchen 1 bis 5, dadurch gekennzeichnet, daß man bei Temperaturen von 190 bis 280°C arbeitet.

7. Verfahren nach den Ansprüchen 1 bis 6, dadurch gekennzeichnet, daß man die Reaktion bei einem Gesamtdruck von 50 bis 200 bar durchführt.

8. Verfahren nach den Ansprüchen 1 bis 7, dadurch gekennzeichnet, daß man einen Katalysator einsetzt, der erhältlich ist durch thermische Zersetzung und Reduktion eines basischen Kupfer-Aluminium-Carbonats, das durch Fällung einer Kupfer- und Aluminiumsalze enthaltenden Lösung bei pH 8 bis 10 gebildet wurde.

**9.** Verfahren nach den Ansprüchen 1 bis 8, dadurch gekennzeichnet, daß man einen Kupferkatalysator verwendet, der durch Erhitzen von Kupferformiat in Gegenwart von Alkohol und Dimethylamin auf Temperaturen oberhalb 170°C erhältlich ist.

**Claims**

**1.** A process for preparing a trialkylamine by reacting a primary alcohol with ammonia or a primary alkylamine in the presence of a hydrogenation/dehydrogenation catalyst and in the presence of hydrogen, which comprises
   a) carrying out the reaction continuously in liquid phase at a total pressure of from 50 to 300 bar,
   b) during addition of the reactant, maintaining a stoichiometric excess of from 1 to 15 moles of the alcohol over the ammonia or primary alkylamine,
   c) carrying out the reaction in the presence of the water formed in the course of the reaction, and
   d) using a hydrogenation/dehydrogenation catalyst which substantially contains only copper as the catalytically hydrogenating/dehydrogenating metal.

**2.** A process as claimed in claim 1, wherein the reactants are reacted in the presence of an alkali metal oxide and/or hydroxide and/or alkaline earth metal oxide and/or hydroxide.

**3.** A process as claimed in claim 2, wherein the alkali metal oxide and/or hydroxide and/or alkaline earth metal oxide and/or hydroxide is used in an amount of from 0.01 to 1, in particular from 0.02 to 0.2, % by weight, based on the amount of reactants.

**4.** A process as claimed in claim 1 or 2 or 3, wherein $C_1$-$C_{24}$-alcohols are used in pure form or in the form of their mixtures.

**5.** A process as claimed in claim 1 or 2 or 3 or 4, wherein the stoichiometric excess of the alcohol over ammonia or the amine ranges from 1.5 to 15, preferably from 2 to 10, moles.

**6.** A process as claimed in claim 1 or 2 or 3 or 4 or 5, wherein the reaction temperature ranges from 190 to 280°C.

**7.** A process as claimed in claim 1 or 2 or 3 or 4 or 5 or 6, wherein the reaction is carried out under a total pressure of from 50 to 200 bar.

**8.** A process as claimed in claim 1 or 2 or 3 or 4 or 5 or 6 or 7, wherein the catalyst used is obtainable by thermal decomposition and reduction of a basic copper aluminium carbonate formed by precipitating a solution containing copper and aluminium salts at pH 8 to 10.

**9.** A process as claimed in claim 1 or 2 or 3 or 4 or 5 or 6 or 7 or 8, wherein the copper catalyst used is obtainable by heating copper formate at above 170°C in the presence of an alcohol and dimethylamine.

**Revendications**

**1.** Procédé de préparation de trialkylamines par la réaction d'alcools primaires avec l'ammoniac ou une alkylamine primaire, en présence d'un catalyseur d'hydrogénation/déshydrogénation, comme aussi en présence d'hydrogène, caractérisé en ce que
   a) on entreprend la réaction en continu et en phase liquide à une pression totale de 50 à 300 bars,
   b) pendant l'addition des réactifs on maintient un excès stoechiométrique de 1 à 15 moles de l'alcool par rapport à l'ammoniac ou à l'alkylamine primaire au cours de la réaction précitée,
   c) on réalise la réaction en présence de l'eau engendrée par la réaction et
   d) on met en oeuvre un catalyseur d'hydrogénation/déshydrogénation qui contient essentiellement du cuivre à titre de métal à activité hydrogénante/déshydrogénante catalytique.

**2.** Procédé suivant la revendication 1, caractérisé en ce que l'on fait réagir les réactifs en présence d'oxydes et/ou d'hydroxydes de métaux alcalins et/ou alcalino-terreux.

3. Procédé suivant la revendication 2, caractérisé en ce que l'on utlise les oxydes et/ou hydroxydes de métaux alcalins et/ou alcalino-terreux en une proportion de 0,01 à 1, plus particulièrement 0,02 à 0,2, % en poids, par rapport à la quantité des réactifs.

4. Procédé suivant les revendications 1 à 3, caractérisé en ce que l'on utilise des alcools en $C_1$-$C_{24}$, sous la forme pure ou sous la forme de leurs mélanges.

5. Procédé suivant les revendications 1 à 4, caractérisé en ce que l'excès stoechiométrique de l'alcool par rapport à l'ammoniac ou à l'amine varie de 1,5 à 15 moles, de préférence 2 à 10 moles.

6. Procédé suivant les revendications 1 à 5, caractérisé en ce que l'on travaille à des températures de 190 à 280°C.

7. Procédé suivant les revendications 1 à 6, caractérisé en ce que l'on entreprend la réaction sous une pression globale de 50 à 200 bars.

8. Procédé suivant les revendications 1 à 7, caractérisé en ce que l'on utilise un catalyseur que l'on peut obtenir par la décomposition thermique et la réduction d'un carbonate de cuivre-aluminium basique, formé par la précipitation d'une solution contenant des sels de cuivre et d'aluminium, à un pH de 8 à 10.

9. Procédé suivant les revendications 1 à 8, caractérisé en ce que l'on utilise un catalyseur au cuivre que l'on obtient par le chauffage de formiate de cuivre en présence d'alcool et de diméthylamine à des températures supérieures à 170°C.